(19) Europäisches Patentamt
European Patent Office
Office européen des brevets

(11) **EP 0 936 563 A2**

(12) **EUROPÄISCHE PATENTANMELDUNG**

(43) Veröffentlichungstag:
18.08.1999 Patentblatt 1999/33

(51) Int. Cl.⁶: **G06F 17/00**

(21) Anmeldenummer: 99102211.2

(22) Anmeldetag: **04.02.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **11.02.1998 DE 19805530**

(71) Anmelder: **Marquette Hellige GmbH**
**79111 Freiburg i. Br. (DE)**

(72) Erfinder:
• **Findeis, Martin**
**79111 Freiburg i. Br. (DE)**

• **Kaiser, Willi**
**79312 Emmendingen (DE)**

(74) Vertreter:
**MÜLLER & HOFFMANN Patentanwälte**
**Innere Wiener Strasse 17**
**81667 München (DE)**

Bemerkungen:
Ein Antrag gemäss Regel 88 EPÜ auf Berichtigung von Schreibfehlern in Beschreibung und Zusammenfassung liegt vor. Über diesen Antrag wird im Laufe des Verfahrens vor der Prüfungsabteilung eine Entscheidung getroffen werden (Richtlinien für die Prüfung im EPA, A-V, 3.).

(54) **Vorrichtung zur Signalverbesserung bei artefaktbehaftetem EKG**

(57) Die Erfindung betrifft eine Vorrichtung zur Signalverbesserung bei artefaktbehaftetem EKG mit einer Mittelwerteinheit (4), die aus einem EKG-Signal den Kurvenverlauf vom Anfang eines QRS-Komplexes bis zum Ende einer T-Welle für eine vorbestimmte Anzahl von Schlägen auswertet und daraus einen Mittelwertschlag bildet. Eine Subtrahiereinheit (3) subtrahiert den Mittelwertschlag von dem EKG-Signal eines aktuellen Schlages, um so ein Restsignal zu erhalten. Eine FIR-Filtereinheit (4) utnerwirft das Restsignal einer Hoch-Tief-Passfilterung und verzögert dieses, um ein gefiltertes Signal zu liefern, zu dem in einer Addiereinheit der Mittelwertschlag addiert wird.

Fig. 1

EP 0 936 563 A2

**Beschreibung**

Die Erfindung betrifft eine Vorrichtung zur Signalverbesserung bei Artefaktbehaftetem Elektrokardiogramm (EKG). Artefakt-behaftete Elektrokardiogramme treten insbesondere bei der Ergometrie, wie beispielsweise der Fahrrad- und Laufbandergometrie, sowie bei der Aufnahme von Langzelt-EKGs auf.

Die bei einem EKG auftretenden Artefakte lassen sich in festfrequente und zufällige Störungen einteilen, die letztgenannten wiederum In die Kategorien Muskelzittern, Bewegungs-Artefakte und Grundlinienschwankungen.

Zur Beseitigung festfrequenter Störungen, wie beispielsweise Netzbrummen, sind verschiedene Methoden bekannt geworden:

So kann beispielsweise ein Netzfrequenz-Kompensationsfilter eingesetzt werden (vgl. JP 1-227 740 A (Abstract)). Es ist aber auch möglich, festfrequente Störungen unter der Voraussetzung zu eliminieren, dass es im Signal Zeiten zwischen den QRS-Komplexen und Zeiten in den QRS-Komplexen gibt und sich Frequenz und Amplitude in diesen Zeiten nicht wesentlich ändern (vgl. US 5,867,735). Schließlich können auch Grundlinienschwankungen und Netzstörungen mit Hilfe von Finite-Impuls-Response-(FIR-)Bandpassfiltern eliminiert werden.

Zufällige Artefakte wie Muskelzittern und Bewegungs-Artefakte werden derzeit durch Tiefpassfilter, wie beispielsweise Filter erster Ordnung mit einer Grenzfrequenz von 20 Hz, reduziert. Solche Tiefpassfilter haben aber nachteilhafte Auswirkungen: sie vermindern insbesondere die Amplituden im QRS-Komplex, so dass diese Amplituden nicht richtig vermessen werden können. Neben der ST-Strecke ist aber auch die R-Amplitude insbesondere für die Beurteilung von Ischämien von großer Bedeutung.

Bekannte Methoden zur Beseitigung von Grundlinienschwankungen sind zum einen Hochpassfilterungen (vgl. US-Z.: IEEE Transactions on Biomedical Engineering, vol. BME-32, 1985, Seiten 1052 - 1060), zum anderen das Spline-Verfahren (C. R. Meyer und H. N. Keiser: "Electrocardiogram baseline noise estimation and removal using cubic splines and state-space computation techniques", Computers and Biomedical Research, vol. 10, Seiten 459 - 470, 1977).

Ein Nachteil von Hochpassfiltern ist die Veränderung der ST-Strecke. Die Messwerte der ST-Strecke, z. B. die ST-Amplitude oder die ST-Steigung, sind für die Beurteilung von Ischämien und die Diagnose der koronaren Herzkrankheit von größter Wichtigkeit. Das Spline-Verfahren führt zu einer unerwünschten Verzögerung des EKGs.

Weiterhin ist eine Vorrichtung zur Verbesserung des Signal-Rausch-Verhältnisses bei einem EKG bekannt (US 5,564,428).

Durch die bekannte Vorrichtung wird das Gesamtsignal gefiltert, wobei für verschiedene Abschnitte des EKGs Filter mit einer Jeweils anderen, für diesen EKG-Abschnitt optimalen Filtercharakteristik verwendet werden. Korrelation und Mittelwertbildung werden eingesetzt, um die jeweils optimalen Filterkoeffizienten zu bestimmen.

Schließlich gibt es noch eine Methode zur Reduzierung des Rechenaufwandes bei der Mustererkennung für eine Arrhythmieanalyse (DE 39 12 028 A1). Diese Methode kann Artefakte für eine ganz bestimmte Art der Signalverarbeitung, nämlich die Arrhythmieanalyse, beseitigen. Sie ist aber nicht geeignet, Artefakte in EKG-Kurven für die Signalspeicherung und -Wiedergabe zu beseitigen.

Für die unmittelbare Beurteilung eines EKGs wird eine möglichst kurze Verzögerungszeit in der Filtereinheit angestrebt, um eine kritische Situation eines Patienten schnell erkennen zu können.

Der Erfindung liegt die Aufgabe zugrunde, eine Vorrichtung zur Signalverbesserung bei einem EKG zu schaffen, mit der das Auftreten von zufälligen Artefakten bei der Signalverarbeitung, -speicherung und -wiedergabe weitgehend reduziert werden kann, ohne das EKG selbst in unzulässiger Weise zu verfälschen oder zu verzögern.

Zur Lösung dieser Aufgabe schafft die Erfindung eine Vorrichtung zur Signalverbesserung bei Artefakt-behaftetem EKG, mit

- einer Mittelwerteinheit, die fortlaufend aus einem EKG-Signal den Kurvenverlauf vom Anfang eines QRS-Komplexes bis zum Ende einer T-Welle von einer vorbestimmten Anzahl von Schlagen auswertet und daraus fortlaufend einen Mittelwertschlag bildet,
- einer Subtrahiereinheit, die den Mittelwertschlag von dem EKG-Signal eines aktuellen Schlages subtrahiert, um ein Restsignal zu erhalten,
- einer Filtereinheit, die das Restsignal einer Hoch-Tief-Passfilterung unterwirft, um ein gefiltertes Signal zu erhalten, und
- einer Addiereinheit, die den Mittelwertschlag wieder zu dem gefilterten Signal addiert.

Bei der erfindungsgemäßen Vorrichtung wird also während einer Ergometrie, wie beispielsweise einer Laufband-Ergometrie, fortlaufend ein Mittelwertschlag durch Mittelwertbildung von beispielsweise sechzehn vorangehenden, gut korrelierenden EKG-Zyklen berechnet. Selbstverständlich können auch mehr oder weniger Zyklen zur Auswertung herangezogen werden.

Der so erhaltene Mittelwertschlag wird sodann von einem EKG-Signal eines aktuellen Schlages subtrahiert, und das so gewonnene Restsignal wird einer Filtereinheit zugeführt, die eine Hoch-Tief-Passfilterung vornimmt. Danach wird der Mittelwertschlag wieder an der Stelle, an der er subtrahiert wurde, zum gefilterten Restsignal addiert.

Durch die fortlaufende Aktualisierung des Mittelwertschlages wird bei biologisch bedingter Veränderung der QRS-Form berücksichtigt, die beispielsweise durch die steigende Belastung bei der Ergometrie entstehen kann. Der Mittelwertschlag repräsentiert das aktuelle EKG.

Das Subtrahieren und Addieren des Mittelwertschlages von dem EKG-Signal eines aktuellen Schlages bzw. zu dem gefilterten Restsignal wird in stellenrichtiger Reihenfolge vorgenommen, so dass der beispielsweise aus sechzehn EKG-Zyklen gewonnene Mittelwertschlag jeweils der Reihe nach vom aktuellen EKG-Signal subtrahiert und zum aktuellen gefilterten Signal addiert wird. Dabei wird aber bei einem EKG-Zyklus lediglich das Segment vom Anfang des QRS-Komplexes bis zum Ende der T-Welle verwendet. Mit anderen Worten, die P-Welle, die dem QRS-Komplex vorausgeht, wird bei der Subtraktion und entsprechend bei der nachfolgenden Addition nicht berücksichtigt. Die Ursache hierfür ist eine unsichere Zuordnung der P-Welle zum QRS-Komplex, was insbesondere für supraventrikuläre Extrasystolen (SES), Vorhofflattern und AV-Blöcke zweiten und dritten Grades gilt.

Die P-Wellen werden also nicht der Signalverbesserung mit der erfindungsgemäßen Vorrichtung unterworfen. Vielmehr können die P-Wellen beispielsweise einer Tiefpassfilterung mit 10 Hz unterzogen werden. Da nämlich in einem Oberflächen-EKG der Frequenzinhalt der P-Wellen gering ist, kann die Wirkung eines Tiefpassfilters von 10 Hz auf die P-Wellen akzeptiert werden.

Auch wird beim Auftreten von ventrikulären Extrasystolen (VES) die Subtraktion des Mittelwertschlages nicht vorgenommen. Vielmehr werden die VES ähnlich wie die P-Wellen einer Tiefpassfilterung mit 10 Hz ausgesetzt. Da nämlich in einem Oberflächen-EKG der Frequenzinhalt der VES reduziert ist, kann die Wirkung der Tiefpassfilterung mit 10 Hz auf die VES akzeptiert werden.

Das EKG-Signal kann aus einer oder mehreren Ableitungen bestehen. Entsprechend besteht dann auch der Mittelwertschlag aus einer oder mehreren Ableitungen.

Mit der erfindungsgemäßen Vorrichtung können auch mehrere verschiedene Mittelwertschläge verarbeitet werden. Die Mittelwerteinheit ist dann in der Lage, einen oder mehrere Mittelwertschläge zu bilden. Die Subtrahiereinheit wählt den am besten passenden Mittelwertschlag aus und subtrahiert diesen vom aktuellen Schlag. Die Addiereinheit addiert dann den von der Subtrahiereinheit ausgewählten Mittelwertschlag.

Wenn mehrere verschiedene Mittelwertschläge verarbeitet werden, können auch ventrikuläre Extrasystolen, die Komplexe eines intermittierenden Schenkelblocks oder pacestimulierte QRS-Komplexe weitere Mittelwertschläge bilden, die in der gleichen Weise behandelt werden wie der erste Mittelwertschlag.

Bei Holter-Systemen kann dann, wenn das gesamte EKG in einem Speicher abgelegt ist, das EKG z. B. zweimal durchgerechnet werden. Beim ersten Durchlauf können die Mittelwertschläge gebildet werden, während beim zweiten Durchlauf dann die Mittelwertschläge vom EKG subtrahiert und zum gefilterten Restsignal addiert werden.

Wie bereits eingangs erwähnt wurde, könnte an sich daran gedacht werden, zur Artefakt-Unterdrückung übliche Tiefpassfilter, wie beispielsweise Filter erster Ordnung mit einer Grenzfrequenz von 20 Hz, zu verwenden. Solche Tiefpassfilter haben aber nachteilhafte Auswirkungen: sie vermindern insbesondere die Amplituden im QRS-Komplex, so dass diese Amplituden nicht richtig vermessen werden können. Neben der ST-Strecke ist aber auch die R-Amplitude insbesondere für die Beurteilung von Ischämien von großer Bedeutung.

Für die unmittelbare Beurteilung eines EKGs wird eine möglichst kurze Verzögerungszeit In der Filtereinheit angestrebt. Diese Verzögerungszeit beträgt bei dem als Filtereinheit eingesetzten Finite-Impuls-Response-(FIR-)Filter lediglich etwa 1 Sekunde.

Für einen einwandfreien Betrieb der erfindungsgemäßen Vorrichtung ist die Korrelation von QRS-Komplexen und damit die Bildung von Mittelwertschlägen Voraussetzung. Ist diese Bildung von Mittelwertschlägen nicht gewährleistet, so schaltet die Filtereinheit automatisch ab, so dass das ungefilterte Signal ausgegeben wird.

Für das Tiefpassfilter der Filtereinheit wird in bevorzugter Weise ein FIR-Filter mit der folgenden Filtergleichung verwendet:

$$y_{n-\frac{N}{2}} = \frac{x_n + x_{n-1} + x_{n-2} + \dots + x_{n-N+1}}{N} \qquad (1)$$

wobei $x_n$, $x_{n-1}$, ... die Darstellung des Eingangssignales,

$$y_{n-\frac{N}{2}}$$

das vom Tiefpassfilter gelieferte Ausgangssignal und N von der Frequenz abhängt und beispielsweise den Wert 24 für ein Tiefpassfilter für 10 Hz hat.

Je nach dem gewünschten Filtereffekt können aber auch kleinere Werte für N eingesetzt werden. Sinnvolle Werte für N bei einer Abtastfrequenz von 500 Hz liegen im Bereich von 6 bis 24. Das entspricht Grenzfrequenzen von ca. 10 Hz bis 40 Hz.

Auch andere Abtastfrequenzen sind möglich.

Die Verzögerung d des Signales ist konstant und gegeben durch:

$$d = \frac{N*T}{2} \tag{2}$$

wobei T das Abtastintervall bedeutet.

Die erforderliche Rechenzeit ist gering, da die obige Gleichung (1) mit

$$S_{n-1} = x_{n-1} + x_{n-2} + \ldots + x_{n-N+1} + x_{n-N} \tag{3}$$

reduziert werden kann zu:

$$y_{n-\frac{N}{2}} = \frac{x_n + S_{n-1} - x_{n-N}}{N} \tag{4}$$

so dass lediglich eine Addition, eine Subtraktion und eine Division auszuführen sind.

Als Hochpassfilter wird für die FIR-Filtereinheit ein Filter mit der folgenden Kennlinie verwendet:

$$y_{n-\frac{N}{2}} = x_{n-\frac{N}{2}} - \frac{x_n + x_{n-1} + x_{n-2} + \ldots + x_{n-N+1}}{N} \tag{5}$$

Für dieses Hochpassfilter gilt beispielsweise N = 500 bei einer Abtastfrequenz von 500 Hz. Je nach dem gewünschten Filtereffekt können auch größere Werte für N eingesetzt werden. Sinnvolle Werte für N bei einer Abtastfrequenz von 500 Hz liegen im Bereich von 500 bis 5000. Das entspricht Grenzfrequenzen von ca. 0,5 Hz bis 0,05 Hz.

Auch andere Abtastfrequenzen sind möglich.

Auch das Hochpassfilter bewirkt eine konstante Verzögerung d des Signales` die in ähnlicher Weise wie in Gleichung (2) gegeben ist durch:

$$d = \frac{N*T}{2} \tag{6}$$

Auch hier ist die erforderliche Rechenzeit gering, da die obige Gleichung (5) mittels der Gleichung:

$$S_{n-1} = x_{n-1} + x_{n-2} + \ldots + x_{n-N+1} + x_{n-N} \tag{7}$$

reduziert werden kann zu:

$$y_{n-\frac{N}{2}} = \frac{x_n + S_{n-1} - x_{n-N}}{N} \tag{8}$$

Das heißt, für die Berechnung des Hochpassfilters sind lediglich eine Addition, zwei Subtraktionen und eine Division auszuführen.

Als konkretes Ausführungsbeispiel der Filtereinheit für eine Abtastrate von beispielsweise 500 Hz kann ein Tiefpassfilter für 10 Hz folgende Filtergleichung haben:

$$y_{n-12} = \frac{x_n + x_{n-1} + x_{n-2} + \ldots + x_{n-23}}{24} \qquad (9)$$

Entsprechend hat ein Hochpassfilter für 0,5 Hz bzw. 0,32 s die folgende Kennlinie:

$$y_{n-250} = x_{n-250} - \frac{x_n + x_{n-1} + x_{n-2} + \ldots + x_{n-499}}{500} \qquad (10)$$

Die erfindungsgemäße Vorrichtung vermag ein Elektrokardiogramm bestehend aus einem oder mehreren Kanälen zu verarbeiten.

Auch ist noch anzumerken, dass die Filtereinheit unabhängig von der Anzahl der Ableitungen arbeitet.

Nachfolgend wird die Erfindung anhand der Zeichnungen näher erläutert. Es zeigen:

Fig. 1             ein Blockschaltbild der erfindungsgemäßen Vorrichtung und

Fig. 2 bis 10      EKG-Diagramme` die veranschaulichen, wie sich eine Mittelwert-(MWS-)Subtraktion, eine Tiefpassfilterung, eine Hochpassfilterung und eine MWS-Addition auf die Signaldarstellung auswirken.

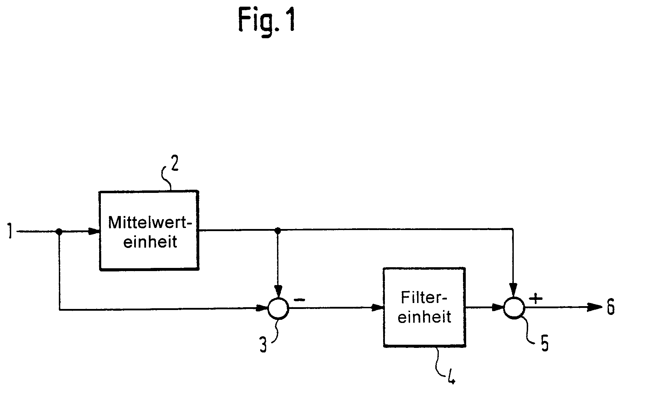

An einem Eingangsanschluss 1 der Vorrichtung der **Fig. 1** liegt ein EKG-Signal, Mittelwerteinheit 2 zugeführt wird, in welcher ein Mittelwertschlag durch Mittelwertbildung aus beispielsweise 16 gut korrelierenden EKG-Zyklen berechnet wird. Dieser Mittelwertschlag wird zu einem Eingangsanschluss eines Subtrahierers 3 gespeist, an dessen anderem Eingangsanschluss das EKG-Signal liegt. Das von dem Subtrahierer 3 erzeugte Rest- bzw. Residuumsignal wird einer Filtereinheit 4 zugeführt, die ein FIR-Hochpassfilter und ein FIR-Tiefpassfilter hat. Nach der Hoch- und Tiefpassfilterung in der Filtereinheit 4 wird das gefilterte Restsignal zu dem Mittelwertschlag in einem Addierer 5 addiert, um so schließlich an einem Ausgang 6 das gefilterte EKG-Signal zu erhalten.

Das Tiefpassfilter und das Hochpassfilter in der Filtereinheit 4 weisen die Filtergleichungen (1) und (5) auf, wie oben erläutert.

Die Verzögerungszeit in der Filtereinheit 4 beträgt lediglich etwa 1 s. eine alleinige Anwendung der FIR-Filtereinheit 4 auf das EKG-Signal vom Eingangsanschluss 1 ohne die Mittelwertschaltung 2 führt dazu, dass die Amplituden des QRS-Komplexes durch das Tiefpassfilter in der Filtereinheit 4 stark reduziert werden. Aus diesem Grund ist die Mlttelwerteinhelt 2, mit deren Hilfe der Mittelwertschlag vor der Filtereinheit 4 subtrahiert und nach der Filtereinheit 4 addiert wird, von besonderem Vorteil.

Die Filtereinheit 4 besteht aus digitalen Filtern, die in bevorzugter Weise in Software realisiert sind. Konkrete Dimensionierungen für diese Filtereinheit 10 sind durch die obigen gleichungen (9) und (10) für eine Abtastrate von 500 Hz gegeben. Gegebenenfalls sind aber auch andere Filter anwendbar, sofern diese eine konstante Verzögerung zu liefern vermögen.

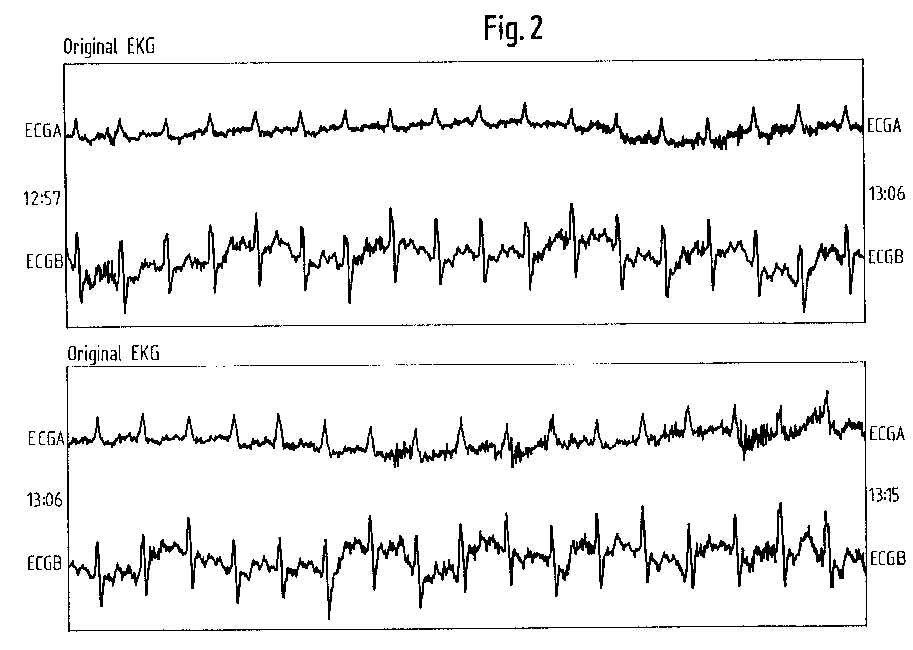

Die Fig. 2 bis 10 zeigen jeweils den gleichen Abschnitt eines EDG-Signals, der verschiedenen Verarbeitungsschritten unterworfen wurde.

**Fig. 2** zeigt das Original-EKG bei dem Rauschen und Grundlinienschwankungen, speziell im zweiten Kananl, deutlich zu erkennen sind.

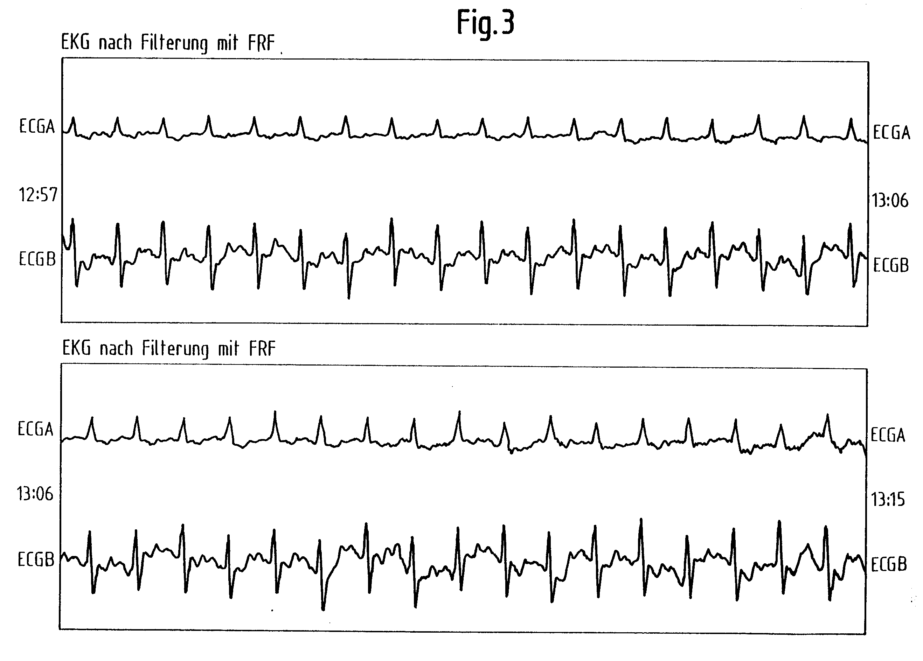

In **Fig. 3** ist der Verlauf des EKG-Signales nach einer Behandlung durch die erfindungsgemäße Vorrichtung und insbesondere nach einer Filterung mit der FRF-Filtereinheit 4 gezeigt: Rauschen und Grundlinienschwankungen sind hier deutlich herausgefiltert.

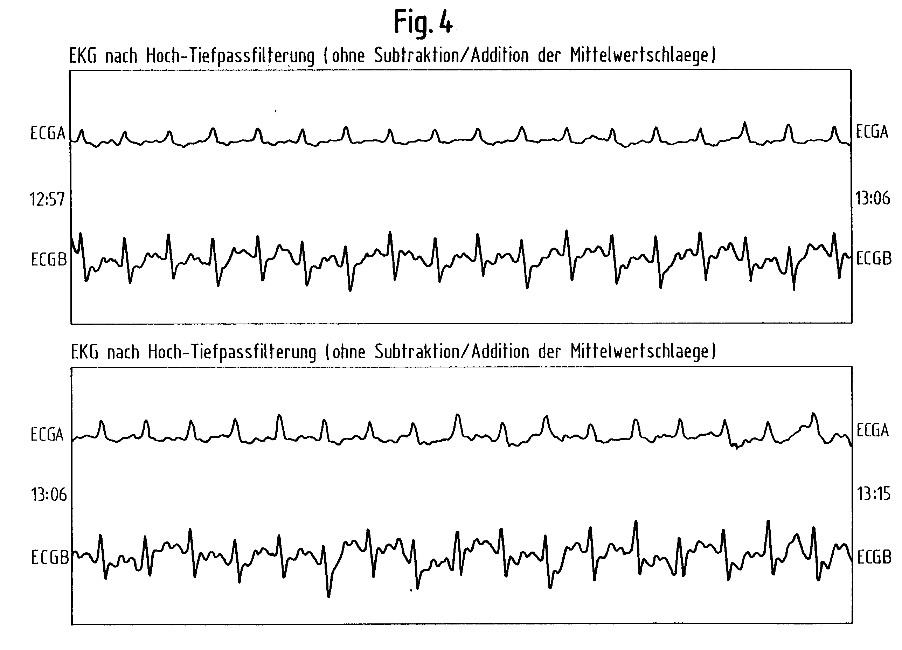

**Fig. 4** zeigt das EKG nach einer Hoch-Tief-Passfilterung, jedoch ohne Subtraktion bzw. Addition des Mittelwertschlages. Hier sind zwar Rauschen und Grundlinienschwankungen herausgefiltert, es treten aber deutliche Amplitudenreduzierungen der QRS-Komplexe auf.

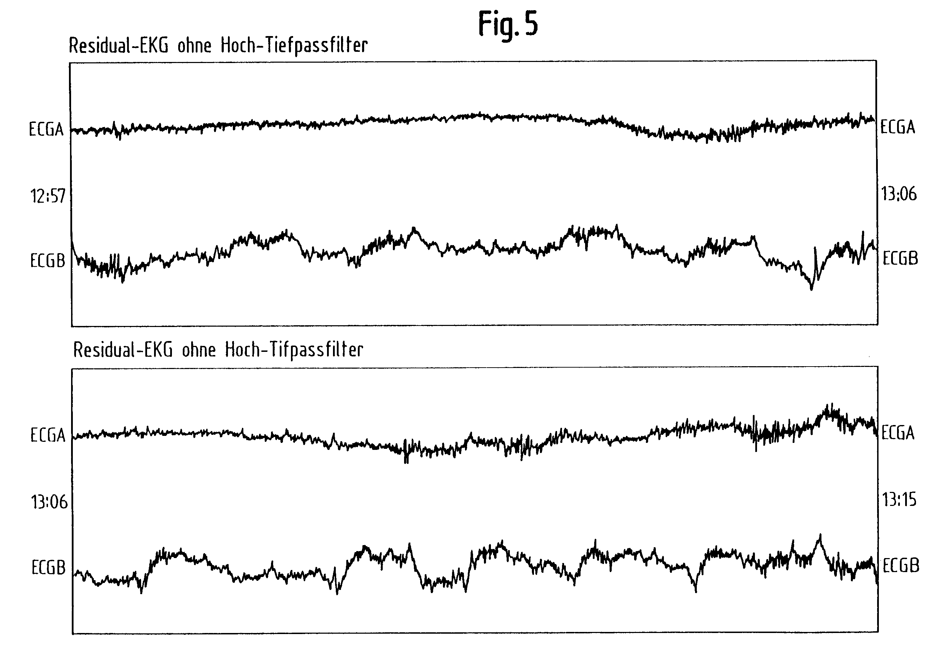

**Fig. 5** zeigt ein Residual-Signal ohne Hoch-Tief-Passfilterung. Rauschen und Grundlinienschwankungen sind hier vorhanden.

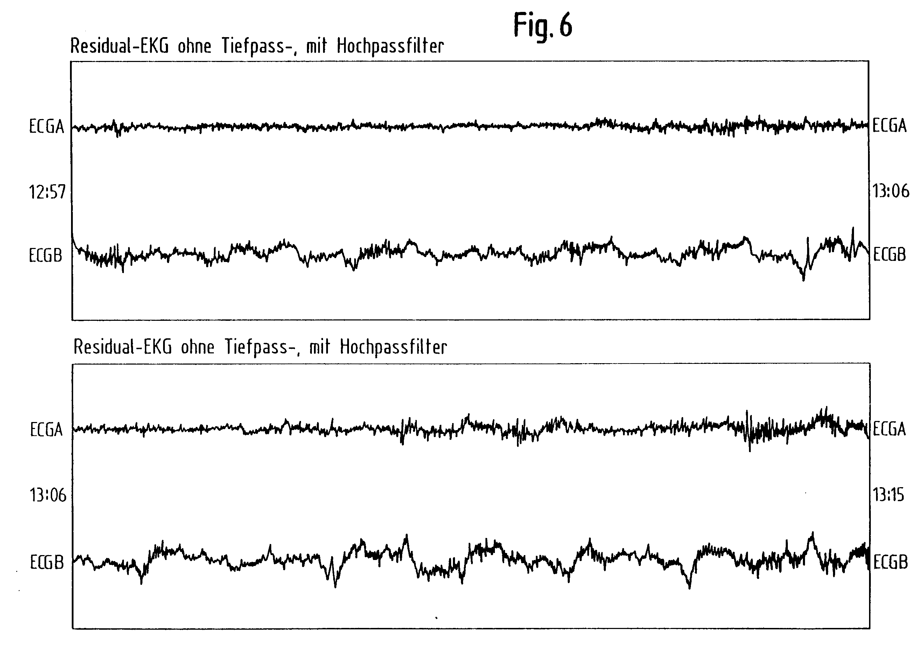

**Fig. 6** zeigt das Residual-Signal ohne Tiefpassfilterung, jedoch mit Hochpassfilterung. Die Grundlinienschwankungen sind hier beseitigt, das Rauschen ist jedoch noch vorhanden.

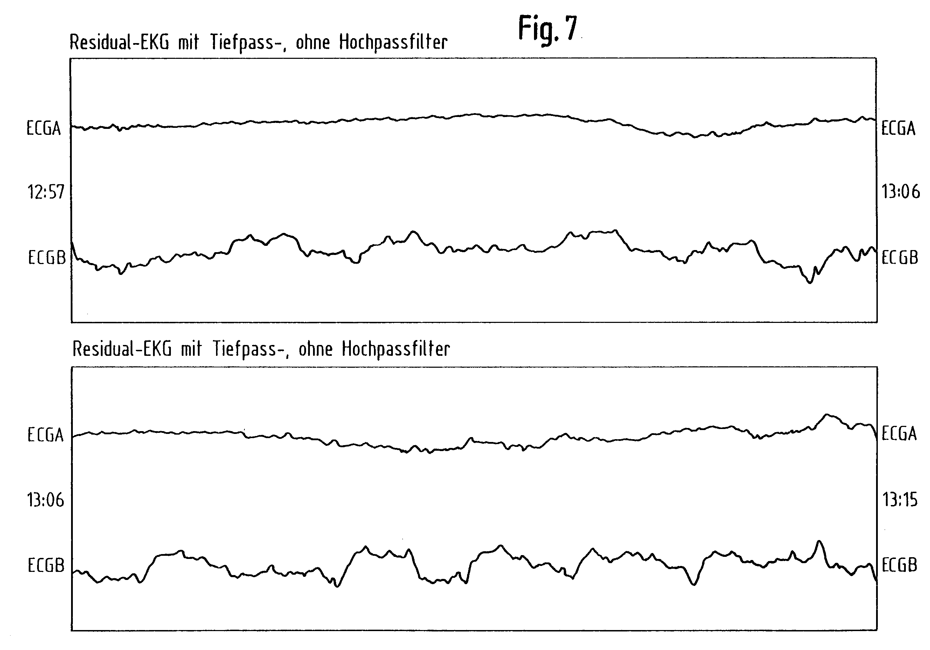

**Fig. 7** zeigt das Residual-Signal mit Tiefpassfilterung, jedoch ohne Hochpassfilterung. Hier ist das Rauschen beseitigt, jedoch sind die Grundlinienschwankungen noch vorhanden.

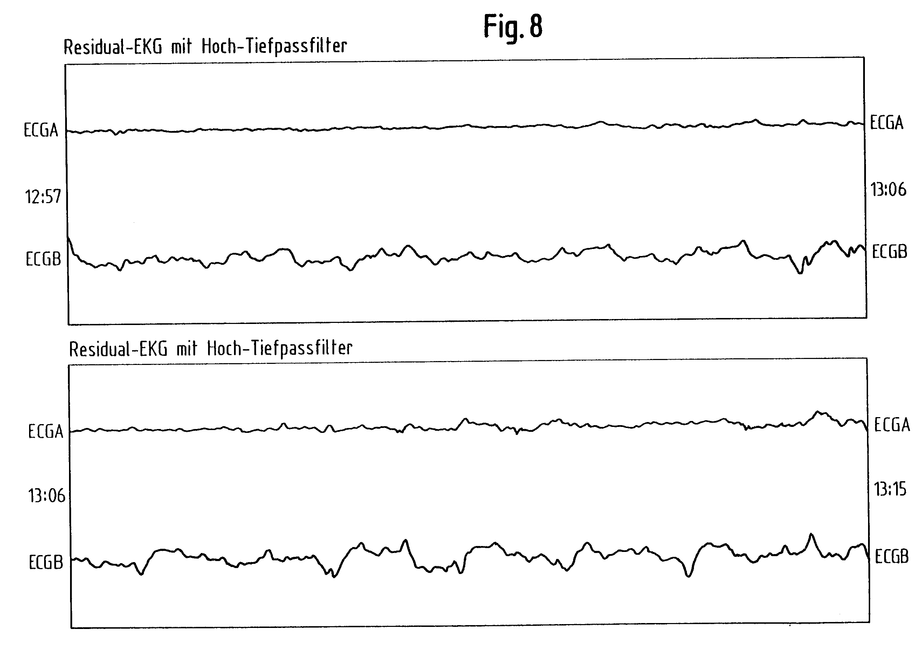

**Fig. 8** stellt das Residual-Signal mit Hoch-Tief-Passfilterung dar. Ähnlich wie in Fig. 3 sind hier Rauschen und Grundlinienschwankungen herausgefiltert.

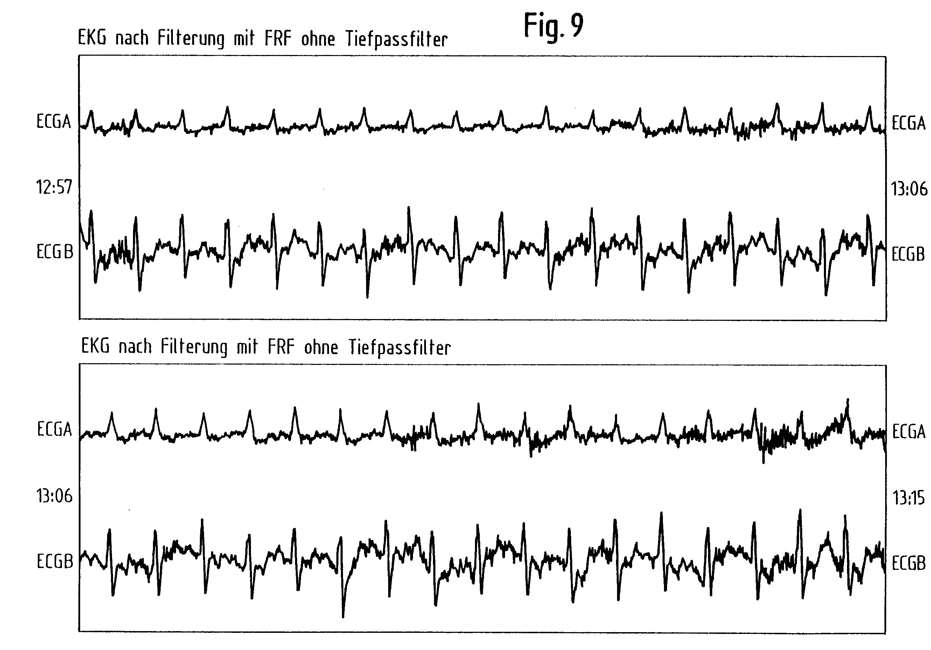

**Fig. 9** zeigt das EDG nach Filterung mit einer FRF-Filtereinheit ohne Tiefpassfilter. Hier sind die Grundlinienschwankungen beseitigt, was aber nicht für das Rauschen gilt.

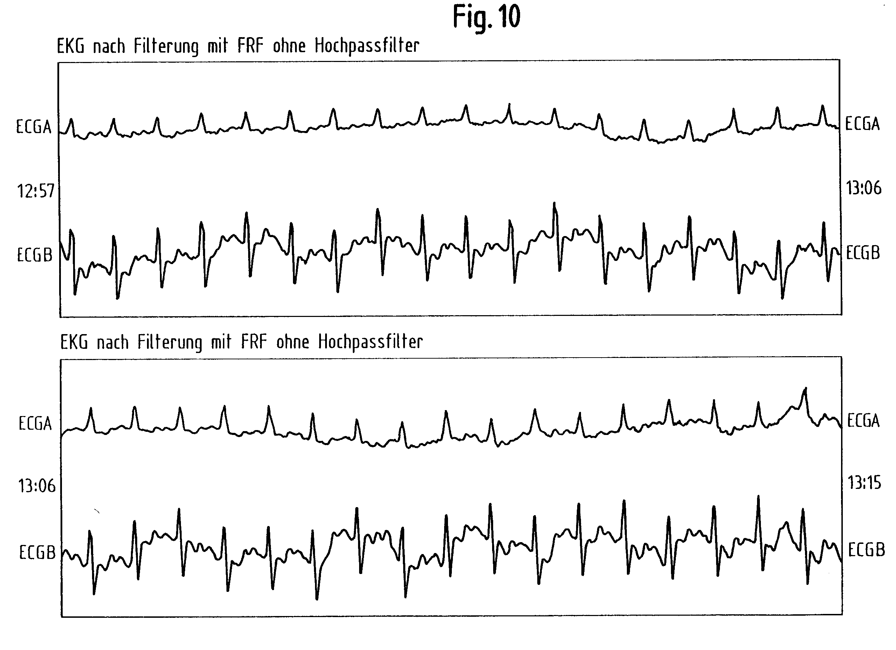

Schließlich zeigt **Fig. 10** ein EKG nach Filterung mit einer FRF-. Filtereinheit ohne Tiefpassfilter. Hier ist das Rauschen beseitigt, jedoch sind die Grundlinienschwankungen noch vorhanden.

In der folgenden Tabelle ist nochmals schematisch angegeben, welche Verarbeitungsschritte bei den betreffenden EKG-Schrieben der Fig. 2 bis 10 vorgenommen wurden.

| Figur | MWS-Subtraktion | Tiefpass-filter | Hochpass-filter | MWS-Addition |
|---|---|---|---|---|
| 2 | - | - | - | - |
| 3 | X | X | X | X |
| 4 | - | X | X | - |
| 5 | X | - | - | - |
| 6 | X | - | X | - |
| 7 | X | X | - | - |
| 8 | X | X | X | - |
| 9 | X | - | X | X |
| 10 | X | X | - | X |

**Patentansprüche**

1. Vorrichtung zur Signalverbesserung bei Artefakt-behaftetem Elektrokardiogramm (EKG), mit:

   - einer Mittelwerteinheit (2), die fortlaufend aus einem EKG-Signal den Kurvenverlauf vom Anfang eines QRS-Komplexes bis zum Ende einer T-Welle von einer vorbestimmten Anzahl von Schlägen auswertet und daraus fortlaufend einen Mittelwertschlag bildet,
   - einer Subtrahiereinheit (3), die den Mittelwertschlag von dem EKG-Signal eines aktuellen Schlages subtrahiert, um ein Restsignal zu erhalten,
   - einer Filtereinheit (4), die das Restsignal einer Hoch-Tief-Passfilterung unterwirft und verzögert, um ein gefiltertes Signal zu erhalten, und
   - einer Addiereinheit (5), die den Mittelwertschlag zu dem gefilterten Signal addiert.

2. Vorrichtung nach Anspruch 1, **dadurch gekennzeichnet,** dass die Subtrahiereinheit (3) den Mittelwertschlag im Bereich vom Anfang des QRS-Komplexes bis zum Ende der T-Welle vom EKG-Signal subtrahiert.

3. Vorrichtung nach Anspruch 1 oder 2, **dadurch gekennzeichnet,** dass die Filtereinheit (4) ein Finite-Impulse-Response-(FIR-)Filter ist.

4. Vorrichtung nach einem der Ansprüche 1 bis 3, **dadurch gekennzeichnet,** dass die Verzögerungszeit der Filtereinheit (4) konstant ist.

5. Vorrichtung nach einem der Ansprüche 1 bis 4, **dadurch gekennzeichnet,** dass die Verzögerungszeit der Filtereinheit (4) etwa 1 s beträgt.

6. Vorrichtung nach Anspruch 3, **dadurch gekennzeichnet,** dass die Filtereinheit (4) ein Tiefpassfilter mit folgender Filtergleichung hat:

$$y_{n-\frac{N}{2}} = \frac{x_n + x_{n-1} + x_{n-2} + \ldots + x_{n-N+1}}{N}$$

mit

N = 24 bei der Abtastrate von 500 Hz,
x = Eingangssignal.
y = Ausgangssignal.

7. Vorrichtung nach einem der Ansprüche 2 bis 5, **dadurch gekennzeichnet,** dass die Filtereinheit (4) ein Hochpassfilter mit folgender Filtergleichung hat:

$$y_{n-\frac{N}{2}} = x_{n-\frac{N}{2}} - \frac{x_n + x_{n-1} + x_{n-2} + \ldots + x_{n-N+1}}{N}$$

mit

N = 500 bei der Abtastrate von 500 Hz,
x = Eingangssignal,
y = Ausgangssignal.

8. Vorrichtung nach Anspruch 3, **dadurch gekennzeichnet,** dass die Filtereinheit (4) ein Tiefpassfilter mit folgender Filtergleichung hat:

$$y_{n-\frac{N}{2}} = \frac{x_n + x_{n-1} + x_{n-2} + \ldots + x_{n-N+1}}{N}$$

mit

$$N = M * \frac{\text{Abtastrate [Hz]}}{500 \text{ Hz}}$$

mit $6 \leq M \leq 24$
x = Eingangssignal,
y = Ausgangssignal.

9. Vorrichtung nach einem der Ansprüche 2 bis 5, **dadurch gekennzeichnet,** dass die Filtereinheit 84) ein Hochpassfilter mit folgender Filtergleichung hat:

$$y_{n-\frac{N}{2}} = x_{n-\frac{N}{2}} - \frac{x_n + x_{n-1} + x_{n-2} + \ldots + x_{n-N+1}}{N}$$

mit

N =

$$M \ * \ \frac{\text{Abtastrate [Hz]}}{500 \text{ Hz}}$$

mit $500 \le M \le 5000$
x = Eingangssignal,
y = Ausgangssignal.

10. Vorrichtung nach einem der Ansprüche 1 bis 9, **dadurch gekennzeichnet,** dass ein Elektrokardiogramm bestehend aus einem oder mehreren Kanälen verarbeitbar ist.

11. Vorrichtung nach Anspruch 1, **dadurch gekennzeichnet,** dass

- die Mittelwerteinheit (2) mehrere verschiedene Mittelwertschläge zu liefern vermag,
- die Subtrahiereinheit (3) den am besten passenden Mittelwertschlag auswählt und ihn von dem EKG-Signal des aktuellen Schlags subtrahiert, und
- die Addiereinheit (5) den von der Subtrahiereinheit (3) ausgewählten Mittelschlag zu dem gefilterten Signal addiert.

# Fig. 1

# Fig. 2

Original EKG

ECGA
12:57
ECGB

ECGA
13:06
ECGB

Original EKG

ECGA
13:06
ECGB

ECGA
13:15
ECGB

EP 0 936 563 A2

## Fig.3

EKG nach Filterung mit FRF

ECGA · 13:06 · ECGB

EKG nach Filterung mit FRF

ECGA · 12:57 · ECGB

ECGA · 13:15 · ECGB

ECGA · 13:06 · ECGB

## Fig. 4.

EKG nach Hoch-Tiefpassfilterung (ohne Subtraktion/Addition der Mittelwertschlaege)

ECGA
12:57
ECGB
ECGA
13:06
ECGB

EKG nach Hoch-Tiefpassfilterung (ohne Subtraktion/Addition der Mittelwertschlaege)

ECGA
13:06
ECGB
ECGA
13:15
ECGB

# Fig. 5

Residual-EKG ohne Hoch-Tiefpassfilter

ECGA — 12:57 — ECGB (left axis); ECGA — 13:06 — ECGB (right axis)

Residual-EKG ohne Hoch-Tifpassfilter

ECGA — 13:06 — ECGB (left axis); ECGA — 13:15 — ECGB (right axis)

Fig.6

## Fig. 7

Residual-EKG mit Tiefpass-, ohne Hochpassfilter

ECGA

12:57

ECGB

ECGA

13:06

ECGB

Residual-EKG mit Tiefpass-, ohne Hochpassfilter

ECGA

13:06

ECGB

ECGA

13:15

ECGB

EP 0 936 563 A2

# Fig. 8

Residual-EKG mit Hoch-Tiefpassfilter

ECGA — ECGA
12:57 — 13:06
ECGB — ECGB

Residual-EKG mit Hoch-Tiefpassfilter

ECGA — ECGA
13:06 — 13:15
ECGB — ECGB

EP 0 936 563 A2

## Fig. 9

EKG nach Filterung mit FRF ohne Tiefpassfilter

EKG nach Filterung mit FRF ohne Tiefpassfilter

EP 0 936 563 A2

# Fig. 10

EKG nach Filterung mit FRF ohne Hochpassfilter

EKG nach Filterung mit FRF ohne Hochpassfilter

EP 0 936 563 A2